# EUROPÄISCHE PATENTSCHRIFT

(11) **EP 0 659 745 B1**
(45) Veröffentlichungstag und Bekanntmachung des Hinweises auf die Patenterteilung: **27.07.2005**
(21) Anmeldenummer: 94119498.7
(22) Anmeldetag: 09.12.1994
(51) Int. Cl.: C07D 231/44, A01N 43/56, A01N 43/40, A01N 43/36, C07D 231/18, C07D 401/04, C07D 403/04, C07D 231/38, C07C 255/30

(54) **Substituierte 1-Arylpyrazole**
Substituted 1-aryl pyrazoles
1-Aryl pyrazoles substitués

(30) Priorität: 22.12.1993 DE 4343832
(43) Veröffentlichungstag der Anmeldung: 28.06.1995
(60) Teilanmeldung: 02024437.2
(73) Patentinhaber: Bayer CropScience AG, 40789 Monheim (DE)
(72) Erfinder: Stetter, Dr. Jörg, D-42115 Wuppertal (DE); Alig, Dr. Bernd, D-53639 Königswinter (DE); Böhm, Dr. Stefan, D-47800 Krefeld (DE); Bertsch, Dr. Achim, D-51061 Köln (DE); Ooms, Dr. Pieter, D-47800 Krefeld (DE); Erdelen, Dr. Christoph, D-42799 Leichlingen (DE); Hartwig, Dr. Jürgen, D-42799 Leichlingen (DE); Wachendorff-Neumann, Dr. Ulrike, D.40789 Monheim (DE); Turberg, Dr. Andreas, D-40699 Erkrath (DE); Mencke, Dr. Norbert, D-51381 Leverkusen (DE)

(56) Entgegenhaltungen:
- EP-A- 0 201 852
- EP-A- 0 295 117
- JOURNAL OF THE CHEMICAL SOCIETY, PERKIN TRANSACTIONS 2, Nr.6, Juni 1993, LETCHWORTH GB Seiten 1099 - 1102 M.J. REMUINAN ET AL. 'Synthesis and Luminiscence Properties of Europium(III) and Terbium(III) Complexes with Polyacid Chelates Derived from 2,6-Bis(N-pyrazolyl)pyridine'
- CHEMICAL ABSTRACTS, vol. 62, no. 12, 7. Juni 1965, Columbus, Ohio, US; 'Sulfonamides.' Spalte 14687C ; & FR-A-1 382 419 (CIBA LTD.) 18. Dezember 1964

## Beschreibung

Die Erfindung betrifft neue substituierte 1-Arylpyrazole, mehrere Verfahren zu ihrer Herstellung und ihre Verwendung als Schädlingsbekämpfungsmittel.

Es ist bereits bekannt, dass bestimmte substituierte 1-Arylpyrazole, wie beispielsweise 5-Amino-1-[2,6-dichlor-4-(trifluormethyl)-phenyl]-3-cyano-4-[(trifluormethyl)-sulfinyl]-1H-pyrazol eine gute Wirksamkeit gegen Schädlinge besitzen (vgl. z.B. EP-A 295 117 und EP-A 352 944).

Weiterhin sind zahlreiche substituierte 1-Arylpyrazole beschrieben, die zur Bekämpfung von Schädlingen eingesetzt werden können (vgl. z.B. EP-A 201 852, EP-A 418 016).

Darüber hinaus dienen substituierte 1-Arylpyrazole auch als Zwischenprodukte für die Herstellung von Schädlingsbekämpfungsmitteln (vgl. z.B. EP-A 301 338, EP-A 301 339, EP-A 374 061, EP-A 260 521).

Die Wirkhöhe bzw. Wirkungsdauer der vorbekannten Verbindungen ist jedoch, insbesondere bei bestimmten Insekten oder bei niedrigen Anwendungskonzentrationen nicht in allen Anwendungsgebieten völlig zufriedenstellend.

Es wurden nun neue substituierte 1-Arylpyrazole der allgemeinen Formel (I) 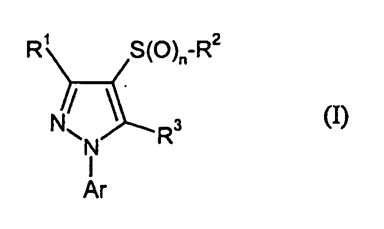 in welcher
- R¹: für Wasserstoff, Cyano, (C₁-C₄)-Alkyl, Methoxymethyl, Ethoxymethyl, Methoxyethyl, Ethoxyethyl, Trifluormethyl, Brommethyl und Cyanomethyl steht,
- R²: für 1,1-Difluorethyl steht,
- R³: für Amino oder für die folgende Gruppierung steht 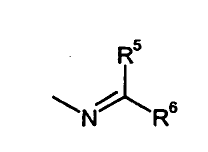
wobei
- R⁵: für Wasserstoff oder (C₁-C₄)-Alkyl steht und
- R⁶: für Phenyl steht,
- Ar: für zweifach oder dreifach, gleich oder verschieden substituiertes Phenyl steht, wobei in der 2-Position Fluor oder Chlor, in der 4-Position Trifluormethyl und in der 6-Position Fluor, Chlor, Cyano, Methoxy, Methylthio, Trifluormethyl, Trifluormethoxy, Trifluormethylthio steht oder
- Ar: für einen 2-Pyridyl-Rest steht, welcher in 5-Position durch Trifluormethyl und in 3-Position durch Fluor oder Chlor substituiert ist und
- n: für eine Zahl 0, 1 oder 2 steht,
gefunden.

Weiterhin wurde gefunden, dass man die neuen substituierten 1-Arylpyrazole der allgemeinen Formel (I) nach einem der im folgenden beschriebenen Verfahren erhält:
a) Man erhält substituierte 1-Aryl-4-mercapto-pyrazole der Formel (Ia), 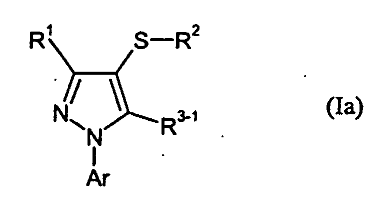 in welcher
   - R¹, R² und Ar: die oben angegebene Bedeutung haben und
   - R³⁻¹: für Amino steht,
   wenn man
   Pyrazolderivate der Formel (II), 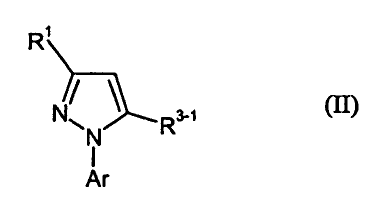 in welcher
   R¹, R³⁻¹ und Ar die oben angegebenen Bedeutungen haben,
   mit Sulfenylhalogeniden der Formel (III)

   R²-S-Hal (III)

   in welcher
   - R²: die oben angegebene Bedeutung hat und
   - Hal: für Halogen, insbesondere Chlor oder Brom steht,
   gegebenenfalls in Gegenwart eines Verdünnungsmittels und gegebenenfalls in Gegenwart eines Reaktionshilfsmittels umsetzt.
b) Man erhält substituierte 1-Arylpyrazole der Formel (Ib), 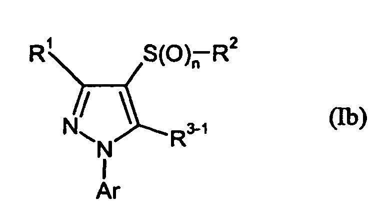 in welcher
   - R¹, R², R³⁻¹ und Ar: die oben angegebenen Bedeutungen haben und
   - n: für die Zahl 1 oder 2 steht,
   wenn man Verbindungen der Formel (Ia) 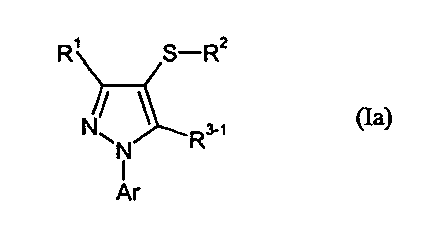 in welcher
   R¹, R², R³⁻¹ und Ar die oben angegebenen Bedeutungen haben
   mit Oxidationsmitteln gegebenenfalls in Gegenwart eines Verdünnungsmittels und gegebenenfalls in Gegenwart eines Katalysators oxidiert.
   Beispielhaft, aber nicht beschränkend sind weitere Herstellungsmethoden für die erfindungsgemäßen Verbindungen der Formel (I) nachstehend aufgeführt, wobei R¹, R², R⁵, R⁶, Ar und n die oben angegebene Bedeutung haben:
c) Umsetzung von substituierten 1-Arylpyrazolen der Formel (Ic) (R³⁻¹ = NH₂) mit Aldehyden oder Ketonen der Formel (VII) 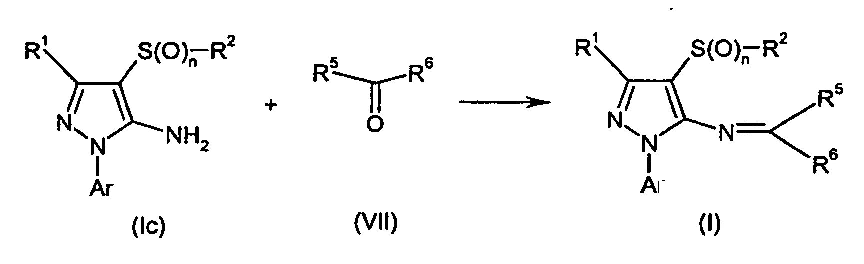
i) Umsetzung von substituierten 1-Arylpyrazolen der Formel (Ic) (R³⁻¹ = NH₂) mit Nukleophilen NU

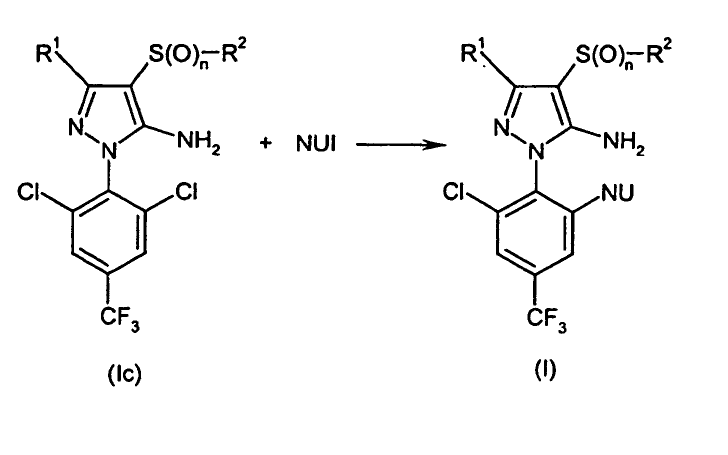

Die oben aufgeführten allgemeinen oder in Vorzugsbereichen aufgeführten Definitionen gelten sowohl für die Endprodukte der Formel (I) als auch entsprechend für die jeweiligen zur Herstellung benötigten Ausgangs- bzw. Zwischenprodukte. Die Definitionen können untereinander, also auch zwischen dem angegebenen bevorzugten Bereich beliebig kombiniert werden.

Die bei der Restdefinition genannten Kohlenwasserstoffreste, wie Alkyl, Alkoxy, Alkoxyalkyl, Alkylthio sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Im einzelnen seien außer den bei den Herstellungsbeispielen genannten Verbindungen die folgenden substituierten 1-Arylpyrazole der allgemeinen Formel (I) genannt:

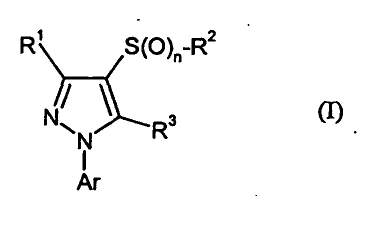

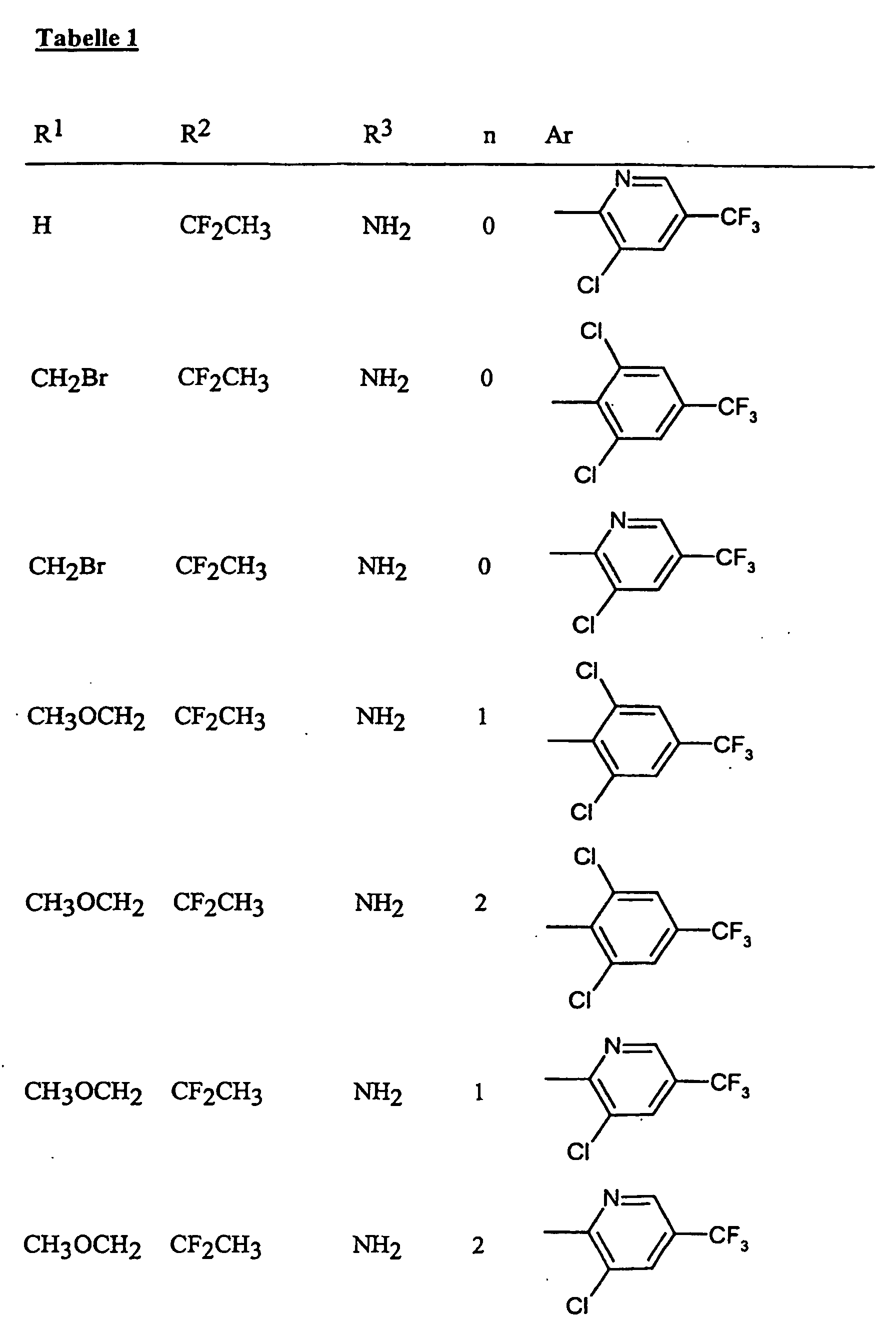

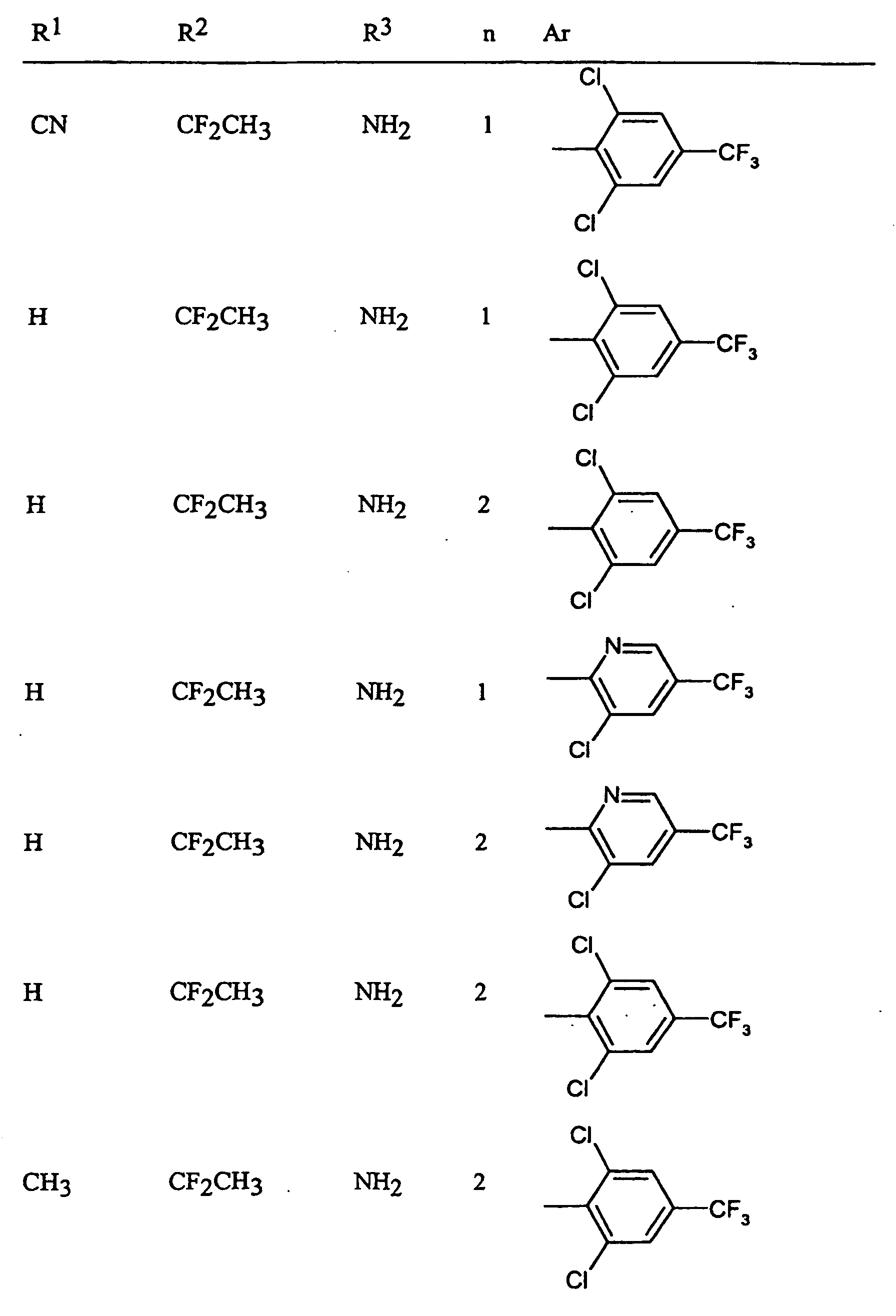

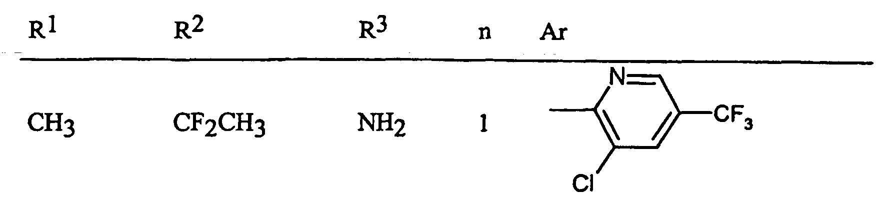

Verwendet man beispielsweise 5-Amino-1-(2,6-dichlor-4-trifluormethylphenyl)-3-methyl-pyrazol und 1,1-Difluorethyl-sulfenchlorid als Ausgangsstoffe, so lässt sich der Reaktionsverlauf des erfindungsgemäßen Verfahrens (a) durch das folgende Formelschema darstellen:

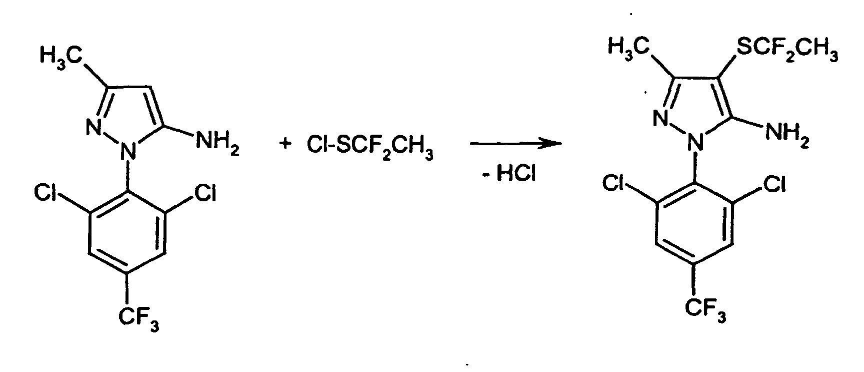

Die zur Durchführung des erfindungsgemäßen Verfahrens (a) als Ausgangsstoffe zu verwendenden Pyrazolderivate der Formel (II) sind teilweise bekannt bzw. können nach bekannten Verfahren erhalten werden (vgl. z.B. EP-A 295 117, EP-A 154 115, EP-A 201 852).

Die Pyrazol-Derivate der Formel (IIa), 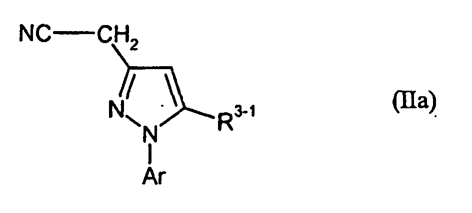 in welcher
- R³⁻¹ und Ar: die oben angegebene Bedeutung haben,
können nach allgemein üblichen und bekannten Verfahren erhalten werden, wenn man Brommethyl-pyrazole der Formel (IIb), 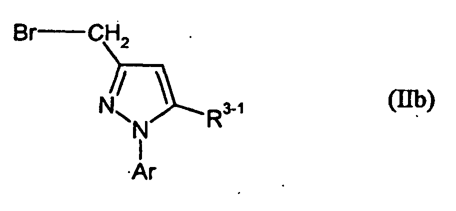 in welcher
- R³⁻¹ und Ar: die oben angegebene Bedeutung haben,
mit Alkalicyaniden, wie beispielsweise Natrium- oder Kaliumcyanid, gegebenenfalls in Gegenwart eines inerten Verdünnungsmittels, wie beispielsweise Wasser und in Gegenwart eines Phasentransferkatalysators, wie beispielsweise TEBA, bei Temperaturen zwischen 40°C und 100°C, vorzugsweise 70°C bis 100°C erhitzt (vgl. Herstellungsbeispiel).

Verwendet man beispielsweise 5-Amino-1-(2,6-dichlor-4-trifluormethylphenyl)-3-brommethyl-pyrazol als Ausgangsstoff, eine wässrige Natriumcyanidlösung und TEBA als Phasentransfer-Katalysator, so lässt sich der Reaktionsablauf des Verfahrens durch das folgende Formelschema darstellen:

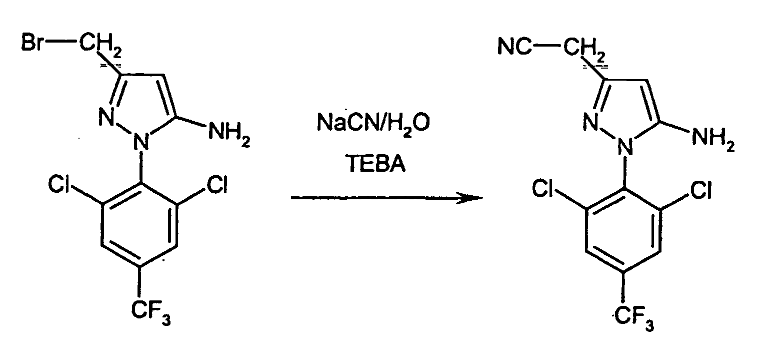

Die Verbindungen der Formel (IIb) werden nach allgemein üblichen und bekannten Verfahren erhalten, wenn man Methoxymethyl-pyrazole der Formel (IIc), 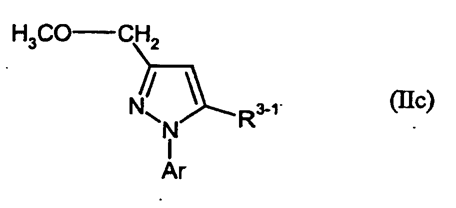 in welcher
- R³⁻¹ und Ar: die oben angegebene Bedeutung haben,
mit einer 48%-igen Bromwasserstoff-Eisessig-Lösung bei Temperaturen zwischen 60°C und 130°C, vorzugsweise bei Temperaturen zwischen 90°C und 130°C, erhitzt (vgl. Herstellungsbeispiele).

Verwendet man beispielsweise 5-Amino-1-(2,6-dichlor-4-trifluormethylphenyl)-3-methoxypyrazol und 48%-ige Bromwasserstoff-Eisessig-Lösung als Ausgangsstoffe, so lässt sich der Reaktionsverlauf des Verfahrens durch das folgende Formelschema darstellen:

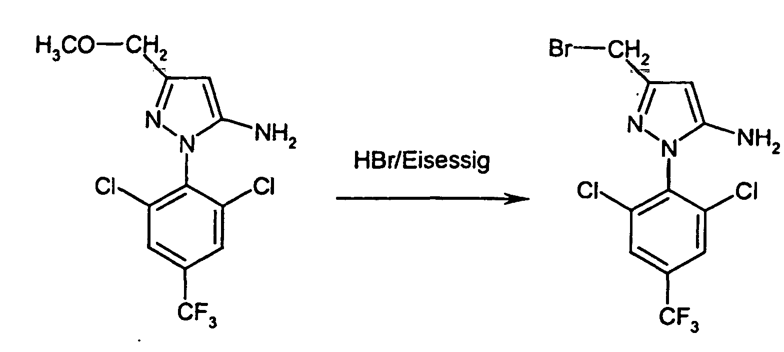

Die Verbindungen der Formel (IIc) können erhalten werden, wenn man Arylhydrazine der Formel (X),

Ar-NHNH₂ (X)

in welcher
- Ar: die oben angegebene Bedeutung hat mit

2-Amino-1-cyano-3-methoxy-propen der Formel (XI) 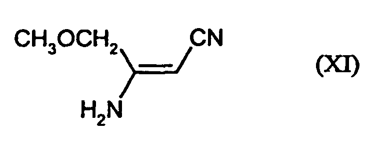 gegebenenfalls in Gegenwart eines inerten organischen Lösungsmittels, wie beispielsweise Alkoholen, vorzugsweise Methanol, Ethanol oder Essigsäure oder Gemische aus Methanol-Essigsäure oder Ethanol-Essigsäure bei Temperaturen zwischen 50°C und 130°C, vorzugsweise 60°C und 120°C erhitzt. Zur Durchführung des Verfahrens setzt man pro Mol Arylhydrazin der Formel (X) im allgemeinen 1 bis 4 Mol, vorzugsweise 1 bis 2 Mol 1-Cyano-2-amino-3-methoxy-propen der Formel (XI) ein. Die Reaktionsführung, Aufarbeitung und Isolierung der Verbindungen der Formel (IVc) erfolgt in üblicher Art und Weise.

Verwendet man als Ausgangsstoffe beispielsweise 2,6-Dichlor-4-trifluormethylphenylhydrazin und 1-Cyano-2-amino-3-methoxy-propen, so lässt sich der Reaktionssverlauf des Verfahrens durch das folgende Formelschema darstellen:

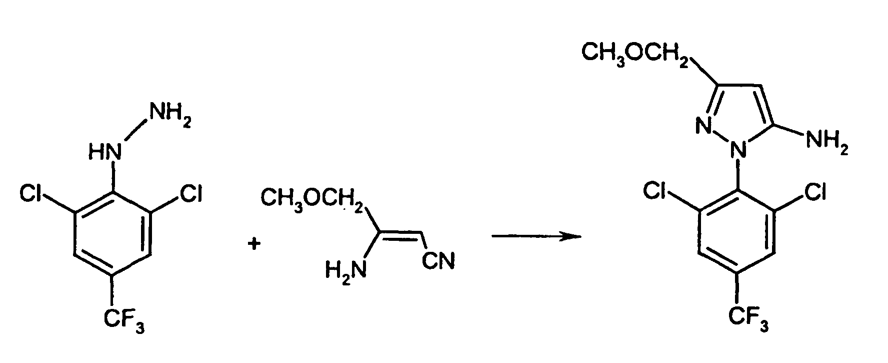

Die als Ausgangsstoffe benötigten Arylhydrazine der Formel (X) sind allgemein bekannte Verbindungen der organischen Chemie.

Man erhält 2-Amino-1-cyano-3-methoxy-propen der Formel (XI), wenn man Methoxyacetonitril der Formel (XII)

CH₃OCH₂-CN (XII)

mit Acetonitril und gegebenenfalls in Gegenwart eines inerten organischen Lösungsmittels, wie beispielsweise Ether, vorzugsweise Diethyl- und Dibutylether, Glykoldimethylether und Diglykolmethylether, Tetrahydrofuran und Dioxan oder aus Gemischen von Acetonitril mit diesen Lösungsmitteln und in Gegenwart von Basen, wie beispielsweise Natriumhydrid oder Kalium-tert-butylat, bei Temperaturen zwischen 20°C und 150°C, vorzugsweise 20°C und 100°C, erhitzt. Zur Durchführung des Verfahrens setzt man im allgemeinen Methoxyacetonitril, die entsprechende Base und Acetonitril in angenähert äquimolaren Mengen ein. Es ist jedoch auch möglich, eine der beiden jeweils eingesetzten Komponenten in einem größeren Überschuss zu verwenden. Die Reaktionsführung, Aufarbeitung und Isolierung der Verbindungen der Formel (XI) erfolgt in üblicher Art und Weise (vgl. Herstellungsbeispiele).

Die Verbindung der Formel (XI) kann als geometrische Isomere (E/Z-Isomere) oder Isomerengemische unterschiedlicher Zusammensetzung vorliegen. Im nachfolgenden wird der Einfachheit halber stets von den Verbindungen der Formeln (XI) gesprochen, obwohl sowohl die reinen Verbindungen als auch ihre Gemische mit unterschiedlichen Anteilen (E/Z-Isomeren) gemeint sind.

Die außerdem zur Durchführung des erfindungsgemäßen Verfahrens (a) als Ausgangsstoffe benötigten Sulfenylhalogenide sind durch die Formel (III) allgemein definiert.

Die Sulfenylhalogenide der Formel (III) sind allgemein bekannte Verbindungen der organischen Chemie.

Die zur Durchführung des erfindungsgemäßen Verfahrens (b) als Ausgangsstoffe benötigten 1-Aryl-4-mercapto-pyrazole sind durch die Formel (Ia) allgemein definiert. In dieser Formel (Ia) stehen R¹, R², R³⁻¹ und Ar vorzugsweise für diejenigen Reste und Indices, die bereits im Zusammenhang mit der Beschreibung der erfindungsgemäßen Stoffe der Formel (I) als bevorzugt für diese Substituenten genannt wurden.

Die Verbindungen der Formel (Ia) sind erhältlich nach Verfahren (a).

Die zur Durchführung der Verfahren (c) und (d) als Ausgangsstoffe benötigten 1-Aryl-4-pyrazole sind durch die Formel (Ic) allgemein definiert. In dieser Formel (Ic) stehen R¹, R², Ar und n vorzugsweise für diejenigen Reste und Indices, die bereits im Zusammenhang mit der Beschreibung der erfindungsgemäßen Stoffe der Formel (I) als bevorzugt für diese Substituenten genannt wurden.

Die Verbindungen der Formel (Ic) sind erhältlich nach Verfahren (a) oder (b).

Die außerdem als Ausgangsverbindungen benötigten Verbindungen der Formel (VII) sind allgemein bekannte Verbindungen der organischen Chemie.

Als Nucleophile (NuI) für die Durchführung des Verfahrens (d) kommen alle üblichen, für derartige Umsetzungen geeigneten Reagenzien der organischen Chemie infrage. Beispielhaft, aber nicht beschränkend seien genannt: Alkoholate, Hydrazinderivate und Cyanide.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril; Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Säuren, wie beispielsweise Essigsäure.

Das erfindungsgemäße Verfahren (a) kann gegebenenfalls in Gegenwart eines Reaktionshilfsmittels durchgeführt werden. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -20°C und + 120°C, vorzugsweise bei Temperaturen zwischen 0°C und +50°C.

Zur Durchführung des erfindungsgemäßen Verfahrens (a) setzt man pro Mol der Pyrazolderivate der Formel (II) im allgemeinen 1,0 bis 2,5 Mol, vorzugsweise 1,0 bis 1,5 Mol an Sulfenylhalogenid der Formel (III) und gegebenenfalls 1,0 bis 2,5 Mol, vorzugsweise 1,0 bis 1,5 Mol an Reaktionshilfsmittel ein. Die Reaktionsführung, Aufarbeitung und Isolierung der Reaktionsprodukte der Formel (Ia) erfolgt nach allgemein üblichen Verfahren.

Als Oxidationsmittel zur Durchführung des erfindungsgemäßen Verfahrens (b) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (b) kommen ebenfalls inerte organische Lösungsmittel infrage. Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolare aprotische Lösungsmittel, wie Acetonitril, Aceton, Essigsäureethylester oder Dimethylformamid.

Das erfindungsgemäße Verfahren (b) kann gegebenenfalls in Gegenwart eines Säurebindemittels durchgeführt werden. Als solche kommen alle üblicherweise verwendbaren organischen und anorganischen Säurebindemittel infrage. Vorzugsweise verwendet man Erdalkali- oder Alkalimetallhydroxide, -acetate oder - carbonate, wie beispielsweise Calciumhydroxid, Natriumhydroxid, Natriumacetat oder Natriumcarbonat.

Das erfindungsgemäße Verfahren (b) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren infrage. Beispielhaft genannt sei in diesem Zusammenhang Ammoniummolybdat und Natriumwolframat.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +70°C, vorzugsweise bei Temperaturen zwischen 0°C und +50°C.

Zur Durchführung des erfindungsgemäßen Verfahrens (b) setzt man pro Mol an substituiertem 1-Arylpyrazol der Formel (Ia) im allgemeinen 0,8 bis 1,2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an substituiertem 1-Arylpyrazol der Formel (Ia) im allgemeinen 1,8 bis 3,0 Mol, vorzugsweise doppelt molare Mengen an Oxidationsmittel ein. Die Reaktionsdurchführung, Aufarbeitung und Isolierung der Endprodukte der Formel (Ib) erfolgt nach üblichen Verfahren.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:
Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion,
Buprofezin, Chlorfluazuron, Diflubenzuron, Flucycloxuron, Flufenoxuron, Hexaflumuron, Pyriproxifen, Tebufenozide, Teflubenzuron, Triflumuron,
Imidacloprid, Nitenpyram, N-[(6-Chloro-3-pyridinyl)methyl]-N'-cyano-N-methylethanimidamid (NI-25),
Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron,
Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox,
Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxsymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität aus.

Die Herstellung der erfindungsgemäßen Verbindungen der Formel (I) soll anhand der folgenden Beispiele erläutert werden:
Prozentangaben beziehen sich, wo nichts anders angegeben wird, auf Gewichtsprozente.

### Herstellungsbeispiele

### Beispiel 1:

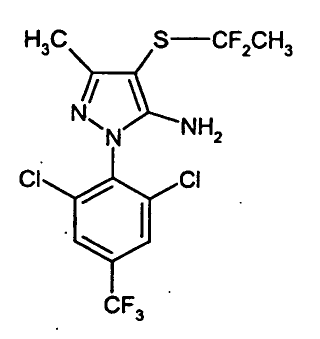

### (Verfahrensvariante a)

15,5g (0,05 Mol) 5-Amino-1-(2,6-dichlor-4-trifluormethylphenyl)-3-methyl-pyrazol werden in 120 ml absolutem Dichlormethan gelöst und mit 4,35g (0,055 Mol) absolutem Pyridin versetzt. Dann kühlt man auf 0-5°C ab und tropft 7,3g (0,055 Mol) 1,1-Difluorethylsulfenchlorid zu. Man rührt 3 Stunden bei 0°C und dann über Nacht bei Raumtemperatur. Anschließend wäscht man 2 mal mit Wasser, trocknet mit Magnesiumsulfat und zieht das Lösungsmittel im Vakuum ab.

Man erhält 13,1g (65 % der Theorie) an 5-Amino-3-methyl-4-(1,1-difluorethylthio)-1-(2,6-dichlor-4-trifluormethylphenyl)-pyrazol vom Schmelzpunkt 123-125°C.

### Beispiel 2:

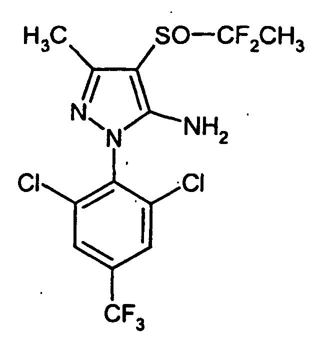

### (Verfahrensvariante b)

4,6g (0,0113 Mol) 5-Amino-3-methyl-4-(1,1-difluorethylthio)-1-(2,6-dichlor-4-trifluormethylphenyl)-pyrazol werden in etwa 10 Minuten bei 25°C-35°C in 30 ml 80%ige Schwefelsäure eingetragen. Unter Kühlung werden 1 ml 35 %ige H₂O₂-Lösung zugetropft, anschließend wird noch 20 Stunden bei Raumtemperatur nachgerührt. Das Reaktionsgemisch wird dann mit Wasser verdünnt und die Ausfällung wird abgesaugt. Nach dem Verrühren mit Petrolether erhält man 1,8g (38 % der Theorie) an 5-Amino-3-methyl-4-(1,1-difluorethylsulfinyl)-1-(2,6-dichlor-4-trifluormethylphenyl)-pyrazol vom Schmelzpunkt 177-179°C.

Analog den Herstellungsbeispielen 1 und 2 und entsprechend den angegebenen Verfahren (a) oder (b) können die folgenden Endprodukte der Formel (I) erhalten werden:

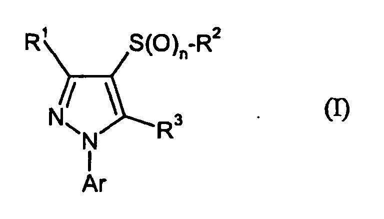

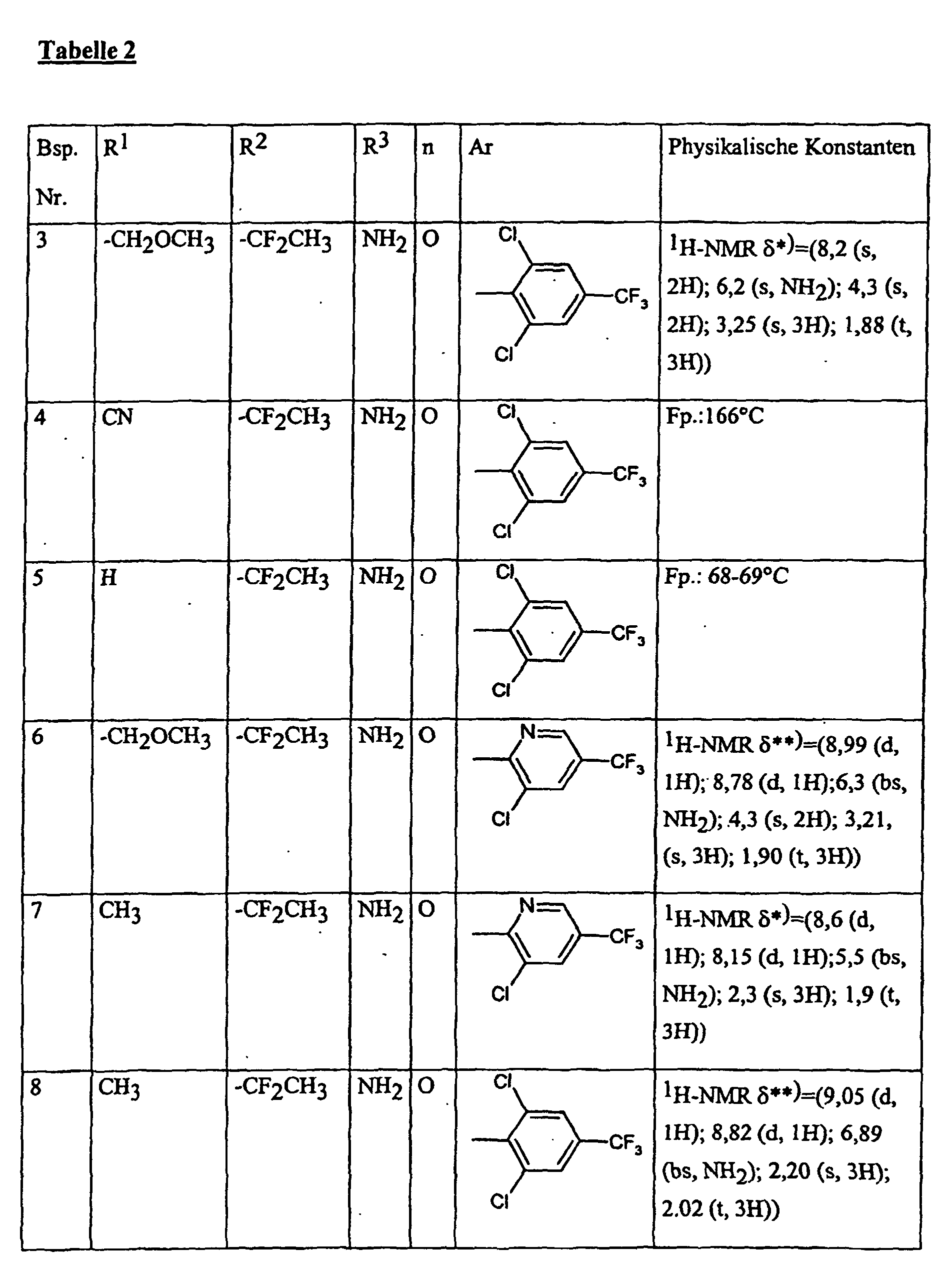

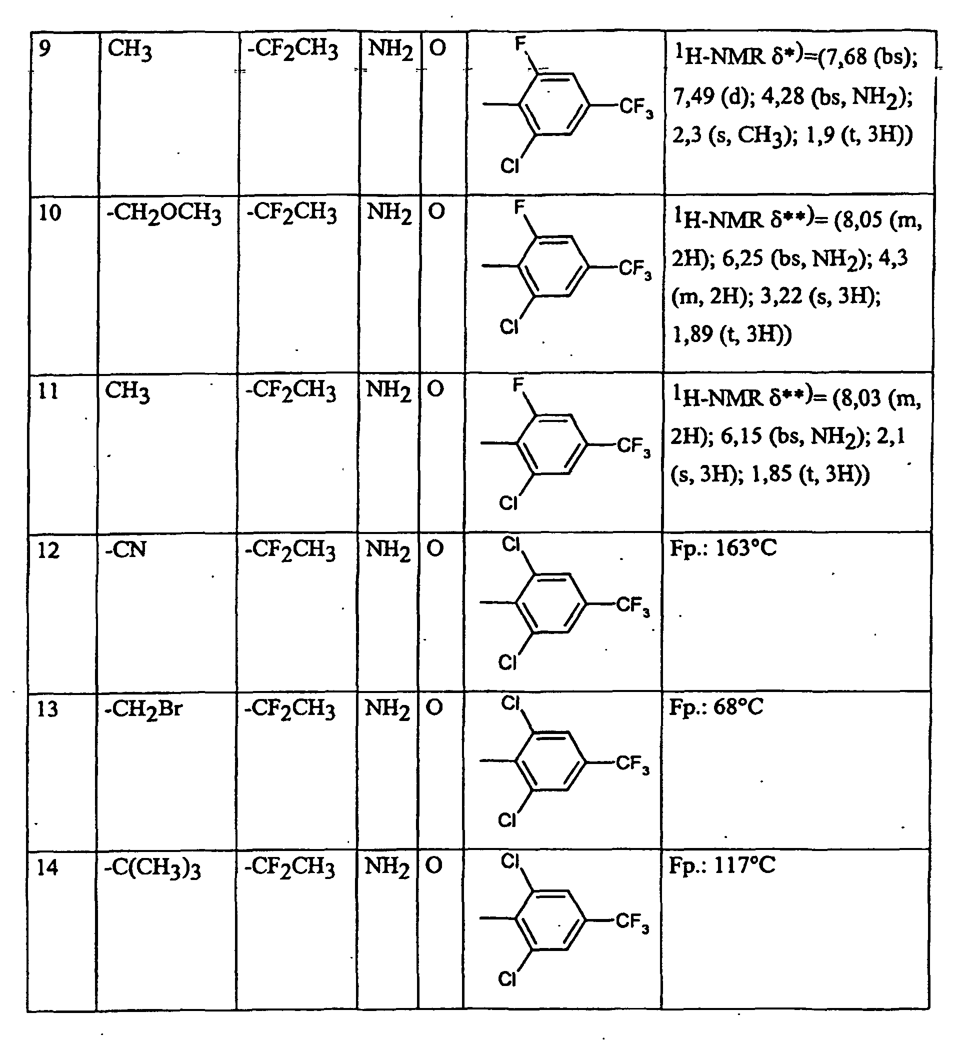

### Beispiel 15

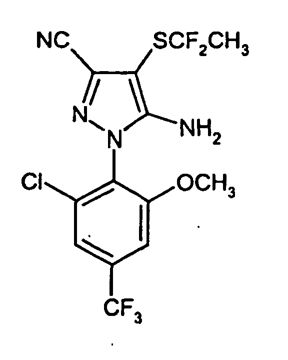

### (Verfahrensvariante d)

1,3g (0,003 Mol) 5-Amino-3-cyano-4-(1,1-difluorethylthio)-1-(2,6-dichlor-4-trifluormethylphenyl)-pyrazol und 0,4g (0,007 Mol) Natriummethylat werden in 20 ml trockenem Methanol 6 Stunden unter Rückfluss erwärmt. Danach werden nochmals 0,3g (0,005 Mol) Natriummethylat zugegeben und weitere 10 Stunden unter Rückfluss erhitzt. Nach dem Abziehen des Lösungsmittels im Vakuum wird der verbleibende Rückstand mit Wasser verrührt und abgesaugt. Der Niederschlag wird mehrmals mit Wasser gewaschen und getrocknet.

Man erhält 1,1g (85 % der Theorie) 5-Amino-3-cyano-4-(1,1-difluorethylthio)-1-(2-chlor-4-trifluormethyl-6-methoxyphenyl)-pyrazol vom Schmelzpunkt 79°C.

### Beispiel 16

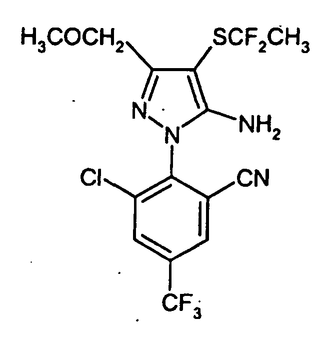

0,7g (1,7 mMol) 5-Amino-1-(2-chlor-6-fluor-4-trifluormethylphenyl)-3-methoxymethyl-4-(1,1-difluorethylthio)-pyrazol und 1g (6,54 mMol) Tetraethylammoniumcyanid werden in 5 ml wasserfreiem Dimethylformamid gelöst und 18 Stunden bei etwa 100-110°C gerührt. Zur Aufarbeitung gießt man auf 100 ml Wasser und saugt den anfallenden Feststoff ab. Nach mehrmaligem Waschen mit Wasser erhält man 0,45 g (63 % der Theorie) an 5-Amino-1-(2-chlor-6-cyano-4-trifluormethyl)-3-methoxymethyl-4-(1,1-difluorethylthio)-pyrazol als ockerfarbenen Feststoff vom Schmelzpunkt 84°C.

### Beispiel 17

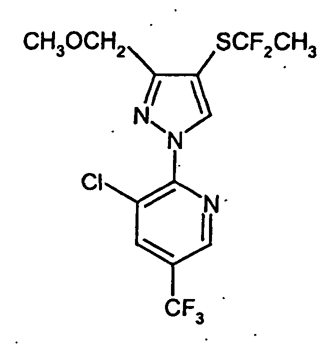

9 g (0,022 Mol) 5-Amino-1-(2-chlor-4-trifluormethylpyridyl)-3-methoxymethyl-4-(1,1-difluorethylthio)-pyrazol werden bei 0°C mit 18 ml Wasser und 18 ml konz. Schwefelsäure versetzt. Bei 0°C wird eine Lösung aus 2,3 g (0,033 Mol) Natriumnitrit und 10 ml Wasser in etwa 30 Minuten zugetropft. Nach Zugabe von 0,5 g Harnstoff werden bei 0°C 27 ml (0,261 Mol) hypophosphorige Säure (50 %ige wässerige Lösung) zugetropft. Danach wird noch 18 h bei Raumtemperatur nachgerührt. Nach der Zugabe von Kaliumcarbonat wird die alkalische Lösung mit Dichlormethan extrahiert. Die vereinigten Dichlormethan-Phasen werden nach dem Trocknen über MgSO₄ im Vakuum eingeengt. Das zurückbleibende Öl wird dann im Kugelrohr destilliert. Bei 180°C/0,1 mm erhält man 4,2 g (48 % der Theorie) 1-(2-Chlor-4-trifluormethylpyridyl)-3-methoxymethyl-4-(1,1,1-difluorethylthio)-pyrazol als orangefarbenes Öl.

Analog den Herstellungsbeispielen 15 bis 17 und entsprechend den angegebenen Verfahren (c) und (d) können die folgenden Endprodukte der Formel (I) erhalten werden:

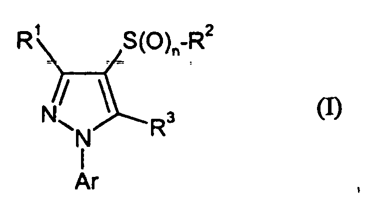

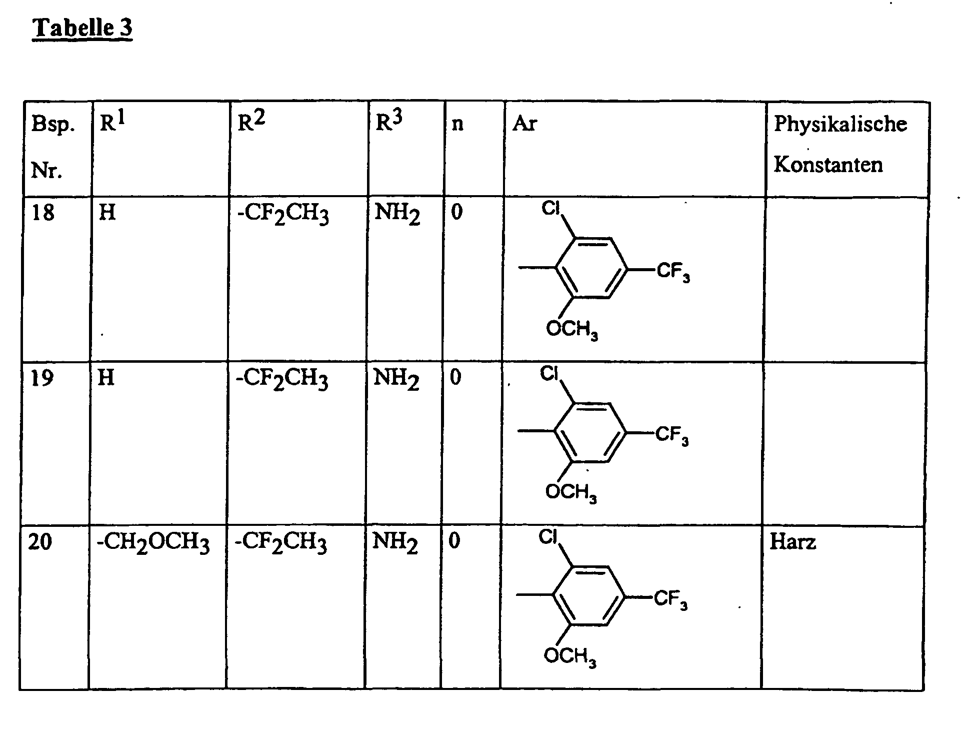

### Herstellung der Ausgangsverbindungen

### Beispiel (II-1)

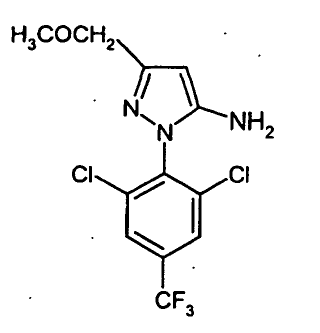

37g (151 mMol) 2,6-Dichlor-4-trifluormethylphenylhydrazin und 4,9g (438 mMol) 1-Cyano-2-amino-3-methoxy-propen in 300 ml Ethanol und 20 ml Essigsäure werden 24 Stunden unter Rückfluss erhitzt. Danach werden nochmals 12g (49 mMol) 2,6-Dichlor-4-trifluormethylphenylhydrazin zugegeben und für weitere 24 Stunden unter Rückfluss erhitzt. Anschließend wird das Lösungsmittel im Vakuum abgezogen, der Rückstand wird säulenchromatographisch (Kieselgel; Laufmittel: Cyclohexan / Essigester 1:1) gereinigt.

Man erhält 48,4g (71 % der Theorie) an 5-Amino-1-(2,6-dichlor-4-trifluormethylphenyl)-3-methoxy-methyl-pyrazol als Öl.

¹H-NMR δ*)= 7,7ppm (s, 2H); 5,74ppm (s, 1H); 4,42ppm (s, 2H); 3,4ppm (s, 3H).

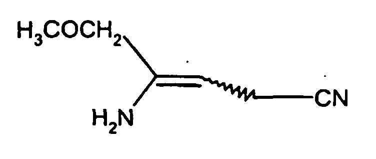

Zu 21,5g (0,19 Mol) Kalium-tert-butylat in 250 ml trockenem Tetrahydrofuran tropft man in etwa 30 Minuten eine Lösung aus 15g (0,21 Mol) Methoxyacetonitril und 10g (0,24 Mol) Acetonitril in 50 ml trockenem Tetrahydrofuran. Nach beendeter Zugabe wird noch 24 Stunden unter Rückfluss erhitzt erhitzt. Nach vorsichtiger Hydrolyse mit Wasser wird mit Dichlormethan extrahiert. Die vereinigten organischen Phasen werden nach dem Trocknen über Magnesiumsulfat im Vakuum eingeengt. Die anschließende fraktionierte Destillation liefert bei 80°C/0,2 mm 8,9g (38 % der Theorie) an 2-Amino-1-cyano-3-methoxypropen als orangefarbenes Öl.

Aus den ¹³C-NMR-Daten ergibt sich ein Verhältnis der E/Z-Isomeren von 1:4.

### Beispiel (II-2):

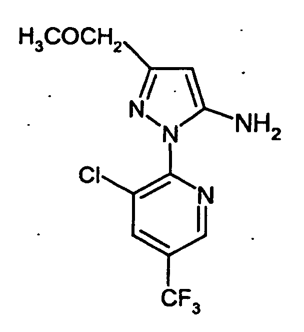

Auf analoge Weise wird aus 2-Chlor-6-fluor-4-trifluormethylphenyl-hydrazin und 2-Amino-1-cyano-3-methoxy-propen 5-Amino-1-(2-chlor-6-fluor-4-trifluormethylphenyl)-3-methoxymethylpyrazol erhalten.

### Beispiel (II-3):

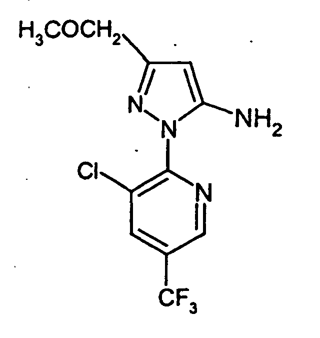

Auf analoge Weise wird aus 2-Chlor-4-trifluormethylpyridyl-hydrazin und 2-Amino-1-cyano-3-methoxypropen 5-Amino-1-(2-chlor-4-trifluormethylpyridyl)-3-methoxymethyl-pyrazol als Öl erhalten.

¹H-NMR δ*) = 8,6ppm (d, 1H); 8,13ppm (d, 1H); 5,67ppm (s, 1H); 4,8ppm (bs, NH₂); 4,41 ppm (s, 2H); 3,4ppm (s, 3H).

### Beispiel (II-4):

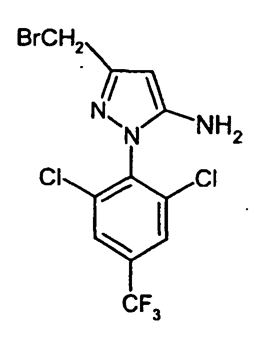

1,5g (4,4 mMol) 5-Amino-1-(2,6-dichlor-4-trifluormethylphenyl)-3-methoxymethyl-pyrazol werden in einer Mischung aus 30 ml 48 %iger HBr und 15 ml Essigsäure etwa 8 Stunden auf 120-130°C erhitzt. Das Reaktionsgemisch wird im Vakuum eingeengt und danach mit verdünnter Ammoniaklösung verrührt. Der braune Feststoff wird abgesaugt und mehrmals mit Wasser gewaschen.

Man erhält 1,6g (93 % der Theorie) 5-Amino-1-(2,6-dichlor-4-trifluormethylphenyl)-3-brommethylpyrazol vom Schmelzpunkt 220°C.

### Beispiel (II-5):

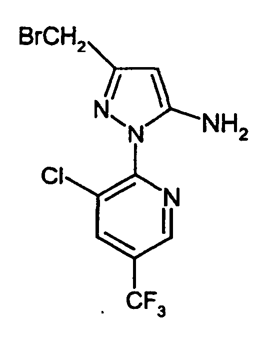

Auf analoge Weise wird das aus 5-Amino-1-(2-chlor-4-trifluormethylpyridyl)-3-methoxymethyl-pyrazol und 48 %iger HBr und Essigsäure 5-Amino-1-(2-chlor-4-trifluormethylpyridyl)-3-brommethyl-pyrazol erhalten.

### Beispiel (II-6):

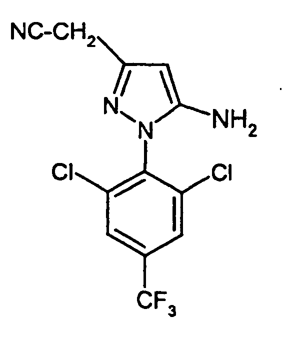

5g (12,9 mMol) 5-Amino-1-(2,6-dichlor-4-trifluormethylphenyl)-3-brommethylpyrazol werden mit 2,5g (51 mMol) Natriumcyanid in 30 ml Wasser in Gegenwart von 4g (17,6 mMol) Triethylbenzylammoniumchlorid 18 Stunden bei 90°C gerührt. Man kühlt auf etwa 5°C ab und saugt den grauen Feststoff ab. Durch Chromatographie an Kieselgel 60 (Laufmittel: Methylenchlorid/Ethanol 1:1) erhält man 1,7g (40 % der Theorie) an 5-Amino-1-(2,6-dichlor-4-trifluormethylphenyl)-3-cyanomethyl-pyrazol als braunen Feststoff vom Schmelzpunkt 98°C.
_{*}) Die ¹H-NMR-Spektren wurden in Deuterochloroform (CDCl₃) mit Tetramethylsilan (TMS) als inneren Standard aufgenommen. Angegeben sind die chemische Verschiebung als δ-Wert in ppm.
_{**}) Die ¹H-NMR-Spektren wurden in deuteriertem Dimethylsulfoxid ((CD₃)₂SO) mit Tetramethylsilan (TMS) als inneren Standard aufgenommen. Angegeben sind die chemische Verschiebung als δ-Wert in ppm.

### Anwendungsbeispiele

In den folgenden Anwendungsbeispielen wird die nachstehend aufgeführte Verbindung als Vergleichssubstanz eingesetzt: 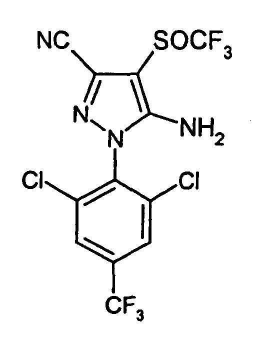 5-Amino-1-[2,6-dichlor-4-(trifluormethyl)-phenyl]-4-[(trimethyl)-sulfinyl]-3-cyano-1H-pyrazol
(bekannt aus EP-A 295 117)

### Beispiel A

| Myzus-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele 1 und 5 überlegene Wirksamkeit gegenüber dem Stand der Technik (Abtötungsgrad 0%), mit einem Abtötungsgrad zwischen 98% und 100% bei einer Wirkstoffkonzentration von 0,1% nach einem Tag.

### Beispiel B

| Aphis-Test (systemische Wirkung) | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so dass die Wirkstoffzubereitung in den Boden eindringt, ohne den Spross zu benetzen. Der Wirkstoff wird von den Wurzeln aufgenommen und in den Spross weitergeleitet.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele 1 und 5 überlegene Wirksamkeit gegenüber dem Stand der Technik (Abtötungsgrad 0%), mit einem Abtötungsgrad zwischen 90 % und 100 % bei gleicher Wirkstoffkonzentration von 0,02 % nach vier Tagen.

### Beispiel C

| Grenzkonzentrations-Test / Wurzelsystemische Wirkung | |
|---|---|
| Testinsekt | Phaedon cochleariae-Larven |
| Lösungsmittel | 3 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alleTesttiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele 1, 5 und 18 überlegene Wirksamkeit gegenüber dem Stand der Technik (Abtötungsgrad 0%), mit einem Abtötungsgrad von jeweils 100 % bei einer Wirkstoffkonzerntration von 2,5 ppm.

### Beispiel D

| Grenzkonzentrations-Test / wurzelsystemische Wirkung | |
|---|---|
| Testinsekt | Myzus persicae |
| Lösungsmittel | 3 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigt z.B. die Verbindung 5 des Herstellungsbeispiels einen Abtötungsgrad von 100 % gegenüber dem Stand der Technik (Abtötungsgrad 0%) bei einer Wirkstoffkonzentration von 20 ppm.

### Beispiel: E

| Blowfly-Larven Test | |
|---|---|
| Testtiere | Lucilia cuprina-Larven |
| Emulgator | 35 Gewichtsteile Ethylenglykolmonomethylether 35 Gewichtsteile Nonylphenolpolyglykolether |

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 24 Stunden wird die Wirksamkeit der Wirkstoffzubereitung ermittelt. Dabei bedeutet 100 %, dass alle Blowfly-Larven abgetötet wurden; 0 % bedeutet, dass keine Blowfly-Larven abgetötet wurden.

Bei diesem Test zeigen z.B. die folgenden Verbindungen der Herstellungsbeispiele 4 und 12 abtötende Wirkung in Höhe von 100 % bei einer Wirkstoffkonzentration ≥ 300 ppm im Vergleich zum Stand der Technik, der diese Wirkung erst bei einer Wirkstoffkonzentration von 1000 ppm erreicht.

### Beispiel F

| Fliegentest | |
|---|---|
| Testtiere | Musca domestica, Stamm WHO (N) |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man drei Gewichtsteile Wirkstoff mit sieben Teilen des oben genannten Lösungsmittel-Emulgator-Gemisches und verdünnt das so erhaltene Emulsionskonzentrat mt Wasser auf die jeweils gewünschte Konzentration.

2 ml dieser Wirkstoffzubereitung werden auf Filterpapierscheiben (⌀ 9,5 cm) pipettiert, die sich in Petrieschalen entsprechender Größe befinden. Nach Trocknung der Filterscheiben werden 25 Testtiere in die Petrieschale überführt und abgedeckt. Nach 6 Stunden wird die Wirksamkeit der Wirkstoffzubereitung ermittelt. Die Wirksamkeit drückt man in % aus. Dabei bedeutet 100 %, dass alle Fliegen abgetötet wurden; 0 % bedeutet, dass keine Fliegen abgetötet wurden.

Bei diesem Test zeigt z.B. die Verbindung des Herstellungsbeispiels 5 eine überlegene Wirkung (100% Abtötung) bei einer Wirkstoffkonzentration von 1 ppm, gegenüber dem Stand der Technik (< 100 %).

### Beispiel G

| Schabentest | |
|---|---|
| Testtiere | Blattella germanica oder Periplaneta americana |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man drei Gewichtsteile Wirkstoff mit sieben Teilen des oben genannten Lösungsmittel-Emulgator-Gemisches und verdünnt das so erhaltene Emulsionskonzentrat mt Wasser auf die jeweils gewünschte Konzentration.

2 ml dieser Wirkstoffzubereitung werden auf Filterpapierscheiben (⌀ 9,5 cm) pipettiert, die sich in Petrieschalen entsprechender Größe befinden. Nach Trocknung der Filterscheiben werden 5 Testtiere bei Blattelle germanica bzw. Periplaneta americana in die Petrieschale überführt und abgedeckt.

Nach 6 Stunden wird die Wirksamkeit der Wirkstoffzubereitung ermittelt. Die Wirksamkeit drückt man in % aus. Dabei bedeutet 100 %, dass alle Fliegen abgetötet wurden; 0 % bedeutet, dass keine Fliegen abgetötet wurden.

Bei diesem Test zeigt z.B. die Verbindung des Herstellungsbeispiels 1 eine überlegene Wirkung (100 % Abtötung >10 ppm Wirkstoffkonzentration) gegenüber dem Stand der Technik (Abtötungsgrad <100 % bei 10 ppm Wirkstoffkonzentration).

### Beispiel H

| In-vitro-Test an Flöhen (alle Entwicklungsstadien) | |
|---|---|
| Testobjekt | Alle Stadien (Eier, Larven, Puppen und Adulte) von Ctenocephalides felis. |
| Testverfahren | Blutmehl wird in einer flachen Schale über Nacht bei ca. 70°C getrocknet und anschließend mit einer Maschenweite von 0,63 mm gesiebt. |
| | Je 1,8g des so aufbereiteten Blutmehls werden in Plastik-Petrischalen von 9,8 cm ⌀ gegeben. |
| | 0,2 ml Substanz werden mit der Eppendorf-Pipette auf die 1,8g Blutmehl gegeben (Verdünnung 1:10). D.h. bei 1 ppm Anwendungskonzentration muss die wässrige Lösung eine Konzentration von 10 ppm haben. Das Aufbringen der Lösung geschieht tropfenweise über die gesamte Oberfläche des Blutmehls verteilt. |
| | Die so vorbereiteten Schalen über Nacht trocknen lassen. Mit einer geeigneten Vorrichtung wird die Substanz, die jetzt in getrockneten Blutmehlklumpen vorliegen, zerstoßen und durch drehende Bewegungen homogen in der Petrischale verteilt. In die so vorbereiteten Testschalen wird nun eine Spatelspitze ausgesiebter Floheier (die von künstlich infizierten Katzen stammen) gegeben. Die Schale wird mit Parafilm verschlossen und kräftig durchgeschüttelt. |
| | Die Inkubation geschieht bei 25°C und 85% relativer Feuchte. Die Schalen werden in bestimmten Zeitabständen auf Entwicklungsstadien der Flöhe untersucht. |
| Testkriterien | Als Kriterien für die in-vitro-Wirkung einer Substanz gilt die Hemmung der Flohentwicklung, bzw. ein Entwicklungsstillstand vor dem Adulten-Stadium. |
| Bewertung | Wirksam: Nach der 1 1/2fachen Entwicklungszeit treten keine adulten Flöhe auf. |
| | Unwirksam Nach der 1 1/2fachen Entwicklungszeit treten adulte Flöhe auf. |

Bei diesem Test zeigt z.B. die Verbindung des Herstellungsbeispiels 4 eine deutlich überlegene Wirkung (100 % Abtötung schon bei 1 ppm Wirkstoffkonzentration) gegenüber dem Stand der Technik (0 % Abtötung bei 10 % Wirkstoffkonzentration).

## Patentansprüche

1. Substituierte 1-Arylpyrazole der allgemeinen Formel (I) 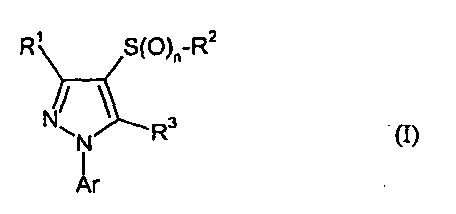 in welcher
R¹ für Wasserstoff, Cyano, (C₁-C₄)-Alkyl, Methoxymethyl, Ethoxymethyl, Methoxyethyl, Ethoxyethyl, Trifluormethyl, Brommethyl und Cyanomethyl steht,
R² für 1,1-Difluorethyl steht,
R³ für Amino oder für die folgende Gruppierung steht 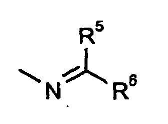
wobei
R⁵ für Wasserstoff oder (C₁-C₄)-Alkyl steht, und
R⁶ für Phenyl steht,
Ar für zweifach oder dreifach, gleich oder verschieden substituiertes Phenyl steht, wobei in der 2-Position Fluor oder Chlor, in der 4-Position Trifluormethyl und in der 6-Position Fluor, Chlor, Cyano, Methoxy, Methylthio, Trifluormethyl, Trifluormethoxy, Trifluormethylthio, steht oder
Ar für einen 2-Pyridyl-Rest steht, welcher in 5-Position durch Trifluormethyl und in 3-Position durch Fluor oder Chlor substituiert ist und
n für eine Zahl 0, 1 oder 2 steht.

2. Verfahren zur Herstellung von substituierten 1-Arylpyrazolen gemäß Anspruch 1, **dadurch gekennzeichnet, daß** man
a) substituierte 1-Aryl-4-mercapto-pyrazole der Formel (Ia) 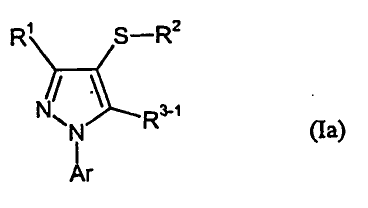 in welcher
R¹, R² und Ar die in Anspruch 1 angegebenen Bedeutungen haben und
R³⁻¹ für Amino steht, erhält,
wenn man
Pyrazolderivate der Formel (II) 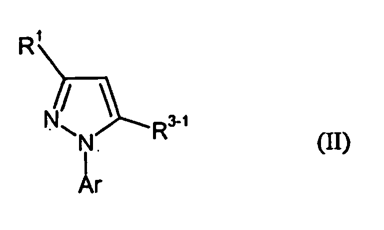 in welcher
R¹, R³⁻¹ und Ar die oben angegebenen Bedeutungen haben,
mit Sulfenylhalogeniden der Formel (III)
R²-S-Hal (III)
in welcher
R² die oben angegebene Bedeutung hat und
Hal für Halogen, insbesondere Chlor oder Brom steht,
gegebenenfalls in Gegenwart eines Verdünnungsmittels und gegebenenfalls in Gegenwart eines Reaktionsmittels umsetzt, oder
b) daß man substituierte 1-Arylpyrazole der Formel (Ib) 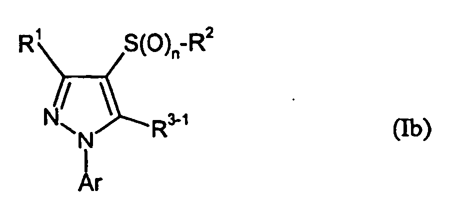 in welcher
R¹, R², R³⁻¹ und Ar die oben angegebenen Bedeutungen haben und
n für die Zahl 1 oder 2 steht, erhält,
wenn man Verbindungen der Formel (Ia) 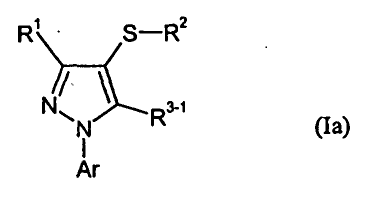 in welcher R¹, R², R³⁻¹ und Ar die oben angegebenen Bedeutungen haben
mit Oxidationsmitteln gegebenenfalls in Gegenwart eines Verdünnungsmittels und gegebenenfalls in Gegenwart eines Katalysators oxidiert.

3. Schädlingsbekämpfungsmittel, **gekennzeichnet durch** einen Gehalt an mindestens einem 1-Arylpyrazol gemäß Anspruch 1.

4. Verfahren zur Bekämpfung von tierischen Schädlingen, insbesondere von Insekten, **dadurch gekennzeichnet, daß** man 1-Arylpyrazole gemäß Anspruch 1 auf tierische Schädlinge und/oder ihren Lebensraum einwirken läßt.

5. Verwendung von 1-Arylpyrazolen gemäß Anspruch 1 zur Bekämpfung von tierischen Schädlingen, insbesondere von Insekten.

6. Insektizide Mittel, **gekennzeichnet durch** einen Gehalt an mindestens einem 1-Arylpyrazol gemäß Anspruch 1.

7. Verfahren zur Herstellung von Mitteln gegen tierische Schädlinge, **dadurch gekennzeichnet, daß** man 1-Arylpyrazole gemäß Anspruch 1 mit Streckmitteln und/oder oberflächenaktiven Mitteln vermischt.

## Claims

1. Substituted 1-arylpyrazoles of the general formula (I) 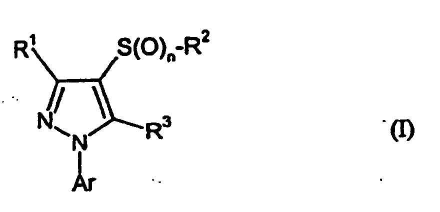 in which
R¹ represents hydrogen, cyano, (C₁-C₄)-alkyl, methoxymethyl, ethoxymethyl, methoxyethyl, ethoxyethyl, trifluoromethyl, bromomethyl and cyanomethyl,
R² represents 1,1-difluoroethyl,
R³ represents amino or the following group 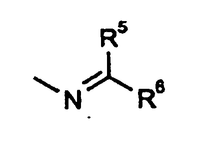
in which
R⁵ represents hydrogen or (C₁-C₄)-alkyl,
R⁶ represents phenyl,
Ar represents phenyl which is disubstituted or trisubstituted by identical or different substituents, where the 2-position is occupied by fluorine or chlorine, the 4-position by trifluoromethyl and the 6-position by fluorine, chlorine, cyano, methoxy, methylthio, trifluoromethyl, trifluoromethoxy, trifluoromethylthio, or
Ar represents a 2-pyridyl radical which is substituted in the 5-position by trifluoromethyl and in the 3-position by fluorine or chlorine, and
n represents a number 0, 1 or 2.

2. Process for the preparation of substituted 1-arylpyrazoles according to Claim 1, **characterized in that**
a) substituted 1-aryl-4-mercapto-pyrazoles of the formula (Ia) 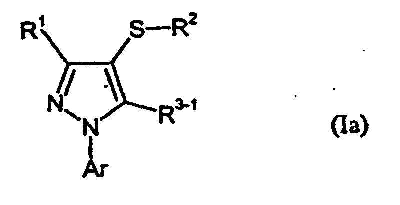 in which
R¹, R² and Ar have the meanings given in Claim 1 and
R³⁻¹ represents amino
are obtained when
pyrazole derivatives of the formula (II) 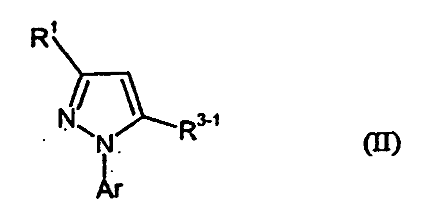 in which
R¹, R³⁻¹ and Ar have the abovementioned meanings,
are reacted with sulphenyl halides of the formula (III)
R²-S-Hal (III)
in which
R² has the abovementioned meaning and
Hal represents halogen, in particular chlorine or bromine,
if appropriate in the presence of a diluent and if appropriate in the presence of a reaction auxiliary, or
b) **in that** substituted 1-arylpyrazoles of the formula (Ib) 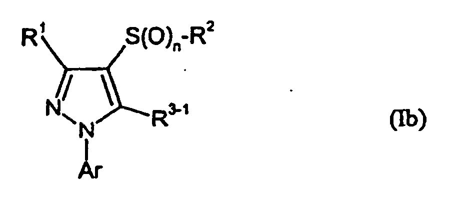 in which
R¹, R², R³⁻¹ and Ar have the abovementioned meanings and
n represents the number 1 or 2
are obtained when compounds of the formula (Ia) 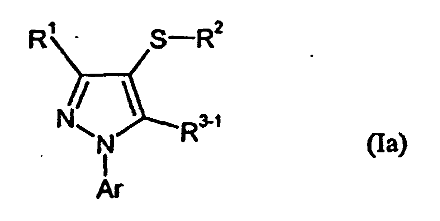 in which
R¹, R² and R³⁻¹ and Ar have the abovementioned meanings
are oxidized using oxidants, if appropriate in the presence of a diluent and if appropriate in the presence of a catalyst.

3. Pesticides, **characterized in that** they contain at least one 1-arylpyrazole according to Claim 1.

4. Method of combating animal pests, in particular insects, **characterized in that** 1-arylpyrazoles according to Claim 1 are allowed to act on animal pests and/or their environment.

5. Use of 1-arylpyrazoles according to Claim 1 for combating animal pests, in particular insects.

6. Insecticidal compositions, **characterized in that** they contain at least one 1-arylpyrazole according to Claim 1.

7. Process for the preparation of compositions against animal pests, **characterized in that** 1-arylpyrazoles according to Claim 1 are mixed with extenders and/or surface-active agents.

## Revendications

1. 1-Arylpyrazoles substitués de la formule générale (I) : 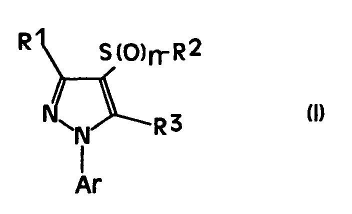 dans laquelle :
R¹ représente l'atome d'hydrogène, le radical cyano, un radical alcoyle (C₁-C₄), les radicaux méthoxyméthyle, éthoxyméthyle, méthoxyéthyle, éthoxyéthyle, trifluorométhyle, bromométhyle et cyanométhyle,
R² représente le radical 1,1-difluoroéthyle,
R³ représente le radical amino ou le groupement suivant : 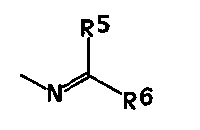
où
R⁵ représente l'atome d'hydrogène ou un radical alcoyle (C₁-C₄),
R⁶ représente le radical phényle ;
Ar représente le radical phényle substitué deux ou trois fois, de manière identique ou différente, où se trouvent en position deux, l'atome de fluor ou de chlore, en position 4, le radical trifluorométhyle et en position 6, l'atome de fluor, de chlore, le radical cyano, méthoxy, méthylthio, trifluorométhyle, trifluorométhoxy, trifluorométhylthio, ou
Ar représente le reste 2-pyridyle, qui est substitué en position 5 par le radical trifluorométhyle et en position 3, par l'atome de fluor ou de chlore, et
n représente le nombre 0, 1 ou 2.

2. Procédé de préparation de 1-arylpyrazoles substitués selon la revendication 1, **caractérisé en ce que** :
a) on obtient les 1-aryl-4-mercaptopyrazoles substitués de la formule (Ia) : 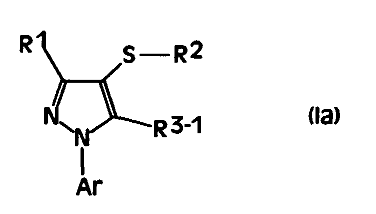 dans laquelle :
R¹, R² et Ar ont les significations indiquées à la revendication 1, et
R³⁻¹ représente le radical amino,
lorsque l'on fait réagir des dérivés de pyrazole de la formule (II) : 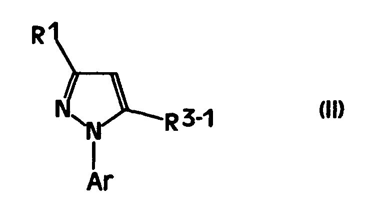 dans laquelle :
R¹, R³⁻¹ et Ar ont les significations indiquées ci-dessus,
avec des halogénures de sulfényle de la formule (III) :
R²-S-Hal (III)
dans laquelle :
R² a la signification indiquée ci-dessus, et
Hal représente un atome d'halogène, en particulier l'atome de chlore ou de brome,
le cas échéant en présence d'un agent de dilution et le cas échéant, en présence d'un auxiliaire de réaction, ou
b) on obtient les 1-arylpyrazoles substitués de la formule (Ib) : 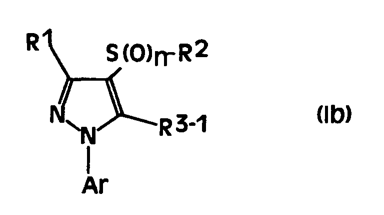 dans laquelle :
R¹, R², R³⁻¹ et Ar ont les significations indiquées ci-dessus, et
n représente le nombre 1 ou 2,
lorsque l'on oxyde les composés de la formule (Ia) : 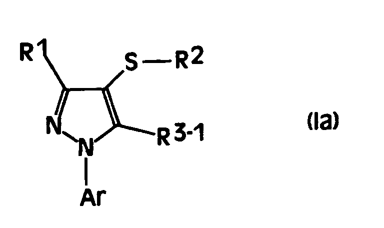 dans laquelle :
R¹, R², R³⁻¹ et Ar ont les significations indiquées ci-dessus,
avec des agents d'oxydation, le cas échéant en présence d'un agent de dilution et le cas échéant, en présence d'un catalyseur.

3. Agent de lutte contre les parasites, **caractérisé par** une teneur en au moins un 1-arylpyrazole selon la revendication 1.

4. Procédé pour lutter contre les parasites animaux, en particulier contre les insectes, qui est **caractérisé en ce que** l'on fait agir des 1-arylpyrazoles selon la revendication 1, sur les parasites animaux et/ou leur biotope.

5. Utilisation de 1-arylpyrazoles selon la revendication 1, pour lutter contre les parasites animaux, en particulier contre les insectes.

6. Agent insecticide, **caractérisé par** une teneur en au moins un 1-arylpyrazole selon la revendication 1.

7. Procédé de préparation d'agents contre les parasites animaux, **caractérisé en ce que** l'on mélange les 1-arylpyrazoles selon la revendication 1, avec des diluants et/ou des agents tensioactifs.
